# EUROPEAN PATENT SPECIFICATION

(11) **EP 3 806 657 B1**
(45) Date of publication and mention of the grant of the patent: **07.08.2024**
(21) Application number: 19819151.2
(22) Date of filing: 13.06.2019
(51) Int. Cl.: A23L 2/60, A23L 27/00, A23L 27/30, A23G 3/34, A23G 9/34

(54) **LOW GLYCEMIC COMPOSITION AND METHODS OF MAKING AND USING THEREOF**
ZUSAMMENSETZUNG MIT NIEDRIGEM GLYKÄMISCHEM INDEX UND VERFAHREN ZUR HERSTELLUNG UND VERWENDUNG DAVON
COMPOSITIONS À FAIBLE INDICE GLYCÉMIQUE ET LEURS PROCÉDÉS DE FABRICATION ET D'UTILISATION

(30) Priority: 14.06.2018 US 201862685066 P
(43) Date of publication of application: 21.04.2021
(73) Proprietor: Seattle Gummy Company, Seattle, Washington 98104 (US)
(72) Inventor: WAN, Feng, Issaquah, Washington 98029 (US); CARLSON, William, Shoreline, Washington 98155 (US); FITCH, Bradley Ryan, Seattle, Washington 98107 (US); LYNN, Kathy, Shoreline, Washington 98155-0014 (US); BAILEY, Christopher David, Seattle, Washington 98115 (US)
(74) Representative: Hobson, David James
(86) International application number: PCT/US2019/037089
(87) International publication number: WO 2019/241583

(56) References cited:
- EP-A1- 3 269 251
- EP-B1- 2 512 270
- EP-B1- 2 945 492
- WO-A1-2011/141129
- WO-A1-2015/006763
- WO-A1-2015/075473
- WO-A1-2016/135458
- WO-A1-2017/059352
- WO-A1-2017/081667
- WO-A1-2017/081667
- WO-A1-2018/029698
- WO-A1-2019/082206
- WO-A1-2019/082206
- WO-A1-2019/140403
- WO-A1-2019/241146
- CA-A- 1 051 260
- US-A1- 2014 342 074
- US-A1- 2014 342 074
- "The technology of wafers and waffles (Volume I) : Operational aspects", 18 May 2017, ELSEVIER, US, article KARL F. TIEFENBACHER: "Technology of Main IngredientsSweeteners and Lipids: 3.2 TECHNOLOGY OF OILS AND FATS FOR WAFERS AND WAFFLES 3.2.1 Introduction", pages: 182 - 215, XP055768404, DOI: 10.1016/B978-0-12-809438-9.00003-X
- ALLULOSE: "Glycemic Index Overview", 1 January 2015 (2015-01-01), pages 1 - 1, XP009525267, Retrieved from the Internet <URL:https://web.archive.org/web/20150812155824/https://allulose.org/allulose-professionals/latest-science/glycemic-index/>
- ANONYMOUS: "Psicose", 3 December 2016 (2016-12-03), pages 1 - 4, XP055770044, Retrieved from the Internet <URL:https://en.wikipedia.org/wiki/Psicose>

## Description

### TECHNICAL FIELD

The application relates generally to food, nutritional, or nutraceutical composition having a low glycemic index.

### BACKGROUND

Current confectioneries and sweet beverage products are flavored with common sugars such as sucrose (table sugar), fructose, and glucose. Sucrose and glucose are simple mono and disaccharides with relatively high glycemic index (GI). For example, sucrose has a GI value of 63 and glucose has GI value of 100. Even though fructose has a relatively low GI value of 19, many recent studies have linked excess fructose with negative health consequences including insulin resistance, fatty liver and increased cholesterol level.

Previous technologies have utilized non-sugar sweeteners (sugar substitutes) to reduce the amount of sugar utilized in confectioneries and sweetened beverages. Some sugar substitutes used are sugar alcohol (such as erythritol, lactitol, maltitol, sorbitol, xylitol, isomalt and mannitol) and non-sugar sweeteners (such as sucralose and aspartame). While these sugar substitutes impart sweetness, they also have "unnatural" sweetness taste, some with unpleasant lingering after taste. For example, sugar alcohols provide sweetness taste that is often described as tacky; and sucralose and aspartame trigger bitter after taste.

In addition, the sugar substitutes are either indigestible or poorly absorbed by the body. Consequently, they change the osmotic pressure within the digestive track. For example, when reaching the colon, sugar substitutes often cause large amount of water to be absorbed into the colon, leading to gastric distress and bloating, gas, and diarrhea.

There is a need for an edible product that does not contain fructose and is low in GI, with a natural sweet taste profile, while not leading to issues such as gastric distress.

WO 2018/029698 A1 describes nutraceutical compositions. EP 2512270 B1 describes a beverage. EP 3269251 A1 describes short texture caramel. WO 2017/081667 A1 describes food and beverage products comprising low calorie, low glycemic index, and a sustained energy release sugar composition. Tiefenbacher (2017), The technology of wafers and waffles Volume 1: Operational aspects, pages 182-215 describes ingredients and their use in wafer and waffle manufacturing. US 2014/342074 A1 describes a sweetener composition for alleviating diabetes, containing a slowly digestible ingredient. WO 2017/059352 A1 describes confections containing allulose. WO 2011/141129 A1 describes a water ice composition. WO 2015/075473 A1 describes food and beverage products comprising allulose. WO 2016/135458 A1 describes allulose syrups. EP 2945492 B1 describes non-cariogenic jelly confectionary. CA 1051260 A describes a sweetening agent. Later-published documents WO 2019/241146 A1, WO 2019/140403 A1, and WO 2019/082206 A1 describe low glycemic gummy compositions, semi-solid anti-histamine compositions, and a sweetness and taste enhancement formulation, respectively.

### SUMMARY

A sweet composition for food application having a low GI value as claimed is provided. The sweet composition may be used for producing food products such as powders, drinks, syrups, bakeries, pastries, gum, evanescent tablets, dairy products, confectionary products, snacks, chewable tablets, etc.

In one embodiment, the sweet composition includes a low GI sugar component having a GI value of not more than 10. The composition comprises at least 50% weight of the low GI sugar component as claimed. The composition is substantially free of D-sucrose, D-fructose, D-glucose, sugar alcohols, and non-sugar sweeteners. In some embodiments, the composition has a sweetness taste profile that is natural and substantially similar to D-sucrose.

In some embodiments, the composition may have a GI value of not more than 15. In some embodiment, the sweet composition may have a GI value from about 0 to about 12.

In some embodiments, the low GI sugars may have a GI value of not more than 15. In some embodiments, the low GI sugars may have a GI value of not more than 12, 10, 8, 5, or 4. In some embodiments, the low GI sugars may have a GI value of about 0. In some embodiments, the low GI sugars may have a GI value of from about 10 to about 12.

The composition comprises at least 50 % weight of low GI sugars. In some embodiments, the composition comprises from about 50% to about 80% weight of low GI sugars. In one embodiment, the composition is substantially free of sugar substitutes. In one embodiment, the composition is substantially free of artificial sweeteners.

The low GI sugar component consists essentially of psicose, isomaltulose, and a third low GI sugar selected from a group consisting of trehalose, sorbose, tagatose, or a combination thereof.

In one embodiment, the low GI sweet composition may include psicose (or allulose) by a weight of not less than 30%, 40%, 50%, 55%, 60%, 70% or 80%. In one embodiment, the low GI sugar component may include psicose by a weight from about 50% to about 60%.

In one embodiment, the low GI sweet composition may include trehalose by a weight of not less than 15%, 20%, 22%, 22.5%, 25%, 30%, 40%, 50%, 55%, or 60%. In one embodiment, the low GI sugar component may include trehalose by a weight from about 20% to about 30%.

In one embodiment, the low GI sweet composition may include isomaltulose by a weight of not less than 5%, 10%, 15%, 20%, 22%, 22.5%, 25%, 30%, 40%, 50%, 55%, or 60%. In one embodiment, the low GI sugar component may include isomaltulose by a weight from about 10% to about 30%.

In one embodiment, the low GI sweet composition may include tagatose by a weight of not less than 1%, 5%, 10%, 15%, 20%, 22%, 22.5%, 25%, 30%, or 40%. In one embodiment, the low GI sugar component may include tagatose by a weight from about 10% to about 30%.

In one embodiment, the low GI sweet composition may include trehalose and isomaltulose in a ratio from about 3:1 to about 1:3. In one embodiment, the low GI sugar component may include trehalose and isomaltulose in a ratio of 4:1, 3:1, 2:1, 1:1, 1:2, 2:1, 3:1, or 1:4 by weight.

In one embodiment, the low GI sweet composition may include trehalose and psicose in a ratio from about 3:1 to about 1:3. In one embodiment, the low GI sugar component may include trehalose and psicose in a ratio of 4:1, 3:1, 2:1, 1:1, 1:2, 2:1, 3:1, or 1:4 by weight.

The low GI sweet composition includes isomaltulose and psicose in a ratio from about 3:1 to about 1:3. In one embodiment, the low GI sugar component may include isomaltulose and psicose in a ratio of 2:1, 1:1, or 1:2 by weight.

In one embodiment, the low GI sweet composition may include psicose and tagatose in a ratio from about 5:1 to about 1:5 by weight. In one embodiment, the low GI sweet composition may include psicose and tagatose of about 3:1 by weight.

In one embodiment, the composition may further comprise glucosamine. In one embodiment, the composition may further include isomalt.

In some embodiments, the low GI sugars may be fully or partially absorbable in the digestive tract. In some embodiments, low GI sugars may digest slowly in the body and have minimal impact on blood sugar levels. In some embodiments, the low GI sugars may be absorbed by the body but are not metabolizes. In some embodiments, the low GI sugars are not readily digested or metabolized by the body. In some embodiments, the low GI sugars are primarily excreted in the urine. As a result, these sugars do not lead to the gastric distress that the sugar alcohols may cause.

Due to surprising results of synergistically enhanced sweetness among the low GI sugars used in the disclosed compositions, the compositions are sweet like conventional sugar composition, often described as having a "natural" or "clean" sweet taste with no unpleasant, lingering or tacky aftertaste. The composition retains a natural flavor and sweetness that the sugar substitutes lack. The panel tasting showed that the low GI compositions have a substantially similar temporal profile and sweetness taste profile to traditional sugars (sucrose, glucose, or fructose).

The sweet compositions can be used for food applications where sweet taste is desired including, without limitation, powdered drink concentrates, powdered flavor pack, sweetened liquid or beverages, syrup, yogurt, ice cream, popsicle, shaved ice, milk or fruit shake, confectionary products, candies, gum, gummies, chocolates, chewable tablets, premixes, etc.

Powdered drink concentrates may be sports drink concentrates, vitamin drink concentrates, fruit drink concentrates, and protein drink concentrates (including, without limitation, milk drinks, soymilk drinks, almond milk drinks, rice milk drinks, chocolate milk drinks, cocoa drinks, etc.)

Powered flavor pack may be a flavor pack used for drinks such as coffee mate or tea flavor pack.

Sweet liquid or beverages may be sports drinks, tea drinks, juices, fruit drinks, coffee drinks, milk drinks, vegetable juice or drinks, pedialytes, nutritional drinks, milk drinks, soymilk drinks, almond milk drinks, rice milk drinks, chocolate milk drinks, cocoa drinks, etc.

The sweet compositions can be used for pharmaceutical applications where sweet taste is desired including, without limitation, powdered drug formula, medicated syrup, powdered drug formula, oral films, chewable tablets, medicated gum, etc.

Powdered drug formula may be a powdered formulation containing active compounds for therapeutic purposes. For example, the active compounds may be vitamins, electrolytes (minerals), anti-inflammatory compounds, pain control compounds, etc.

Syrup may be any sweetened syrup for flavoring or medicated syrup for disease treatment including without limitation syrup for drinks, cough syrup, etc. Confectionary products can be any candies or sweets. Premixes may be cake mix, brownies mix, cocoa mix, grain mix (for drink or porridges), etc.

The application further provides a sweet composition as claimed having the low GI sugar component and a carbonation agent comprising carbonate, bicarbonate or a combination thereof.

The application further provides a powdered drink concentrate, comprising the low GI sweet composition as claimed. In one embodiment, the powdered drink concentrate may further comprise a vitamin composition, a mineral composition, an herbal composition, a fruit extract, a carbonation agent, an antioxidant, a food acid, a dispersant, a flavoring agent, a coloring agent, or a combination thereof.

The application further provides a syrup, comprising not less than 50% of the sweet composition as claimed. In one embodiment, the syrup may further comprise an active pharmaceutical ingredient, a flavoring agent, a coloring agent, or a combination thereof.

The application further provides a confectionary product, comprising not less than 50% of the sweet composition as claimed.

The application further provides a liquid composition, comprising not less than 5% of the sweet composition as claimed. The liquid composition may further comprise a vitamin composition, a mineral composition, an Active Pharmaceutical Ingredient (API), or a combination thereof.

The application further provides a gum or chewable tablet, comprising the sweet composition as claimed. The gum or chewable tablet may further comprise nicotine or caffeine.

The application further provides a food product, comprising the sweet composition as claimed. The food product may be popsicle, an ice cream, a milk product, a milk shake, a fruit juice, a coffee product, a soy milk product, yogurt, frozen yogurt, a slurpee, a liquid drink, a coffee mate, a carbonated drink, a soft drink, a soda, or a cooking mix.

The sweet compositions disclosed herein are safer to diabetics to consume due to its low GI value. The low GI sugars may lead to the composition properties such as, without limitation, low GI, increasing metabolism, helping to maintain joint health, reducing tooth decay and helping to reduce rates of obesity. Some embodiments may have anti-hyperglycemic and antihyperlipidemic properties. In one embodiment, the claimed sweet composition may be used by for obese and diabetic subjects.

### BRIEF DESCRIPTION OF THE DRAWINGS

The foregoing and other features of this disclosure will become more fully apparent from the following description and appended claims, taken in conjunction with the accompanying drawings. Understanding that these drawings depict only several embodiments arranged in accordance with the disclosure and are, therefore, not to be considered limiting of its scope, the disclosure will be described with additional specificity and detail through use of the accompanying drawings, in which:
FIGURE 1 shows the chemical structures for psicose, sorbose, and tagatose and their structural relation to fructose;
FIGURE 2 shows the GI and calorie per gram of tagatose;
FIGURE 3 shows that trehalose digests and releases glucose very slowly compared to most sugars; and
FIGURE 4 shows that isomaltulose digests very slowly and has very low impact on blood glucose levels.

### DETAILED DESCRIPTION

Sugar is a broad term chemically and is often used interchangeably with carbohydrate. Common sugars used often in food applications include glucose, sucrose (table sugar), lactose (milk sugar), or fructose (fruit sugar). Not all sugars are sweet. Simple sugars such as sucrose and fructose are sweet. Mannose is bitter while raffinose has no taste to it. Lactose has very little sweetness; and fructose can be twice as sweet as table sugar.

The application provides a low GI sweet composition as claimed for, among others, food application. The composition has a low GI and, therefore, is safe for diabetics to use. In one embodiment, the composition has a GI value of not exceeding 15, 12, 10, or 8. In one embodiment, the sweet composition has a GI value of not exceeding 18 and is substantially free of fructose.

The composition comprises low GI sugars as claimed. "Low GI sugar" in this disclosure refers to non-fructose simple sugars having a GI value of not exceeding 30. In one embodiment, the low GI sugar has a GI value not exceeding 18. In one embodiment, the low GI sugar has a GI value not exceeding 12. In one embodiment, the composition comprises not less than 60% of low GI sugars.

In one embodiment, the compositions may increase metabolism and promote weight loss. In one embodiment, the sweet compositions, unlike traditional sugar-alcohol based "sugar free" products, may prevent or be void of gastric distress. In one embodiment, the sweet compositions have a sweetness taste profile and temporal profile substantially similar to table sugar (sucrose). In some embodiments, the sweet compositions have a sweet and good tasting like a "conventional" sugar containing products, but with the health benefits of prebiotics and lack of negatives that "conventional" sugar containing products have, such as high GI.

In one embodiment, the composition further comprises flavoring agent. In one embodiment, the sweet composition further comprises coloring agent. In one embodiment, the sweet composition further comprises an active component.

### Low GI sugar component

### Monosaccharides

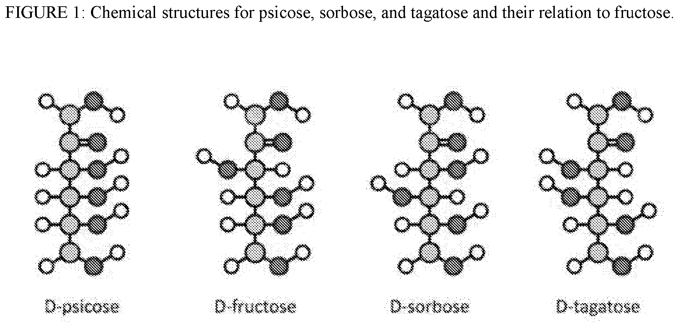

Psicose, sorbose, and tagatose are mono-saccharides and are isomers of D-fructose. They are ketoses and are C3-C5 stereoisomers of fructose. Psicose, sorbose, and tagatose are sugars (carbohydrates) like sucrose or glucose mentioned above. Psicose is identical to sucrose (table sugar) in sweetness but has nearly zero calories and does not promote tooth decay. Tagatose is nearly as sweet as sucrose yet only has 38% of the caloric value of sucrose and is much more tooth friendly than sucrose. Sorbose is equivalent to sucrose in sweetness. Since these are sugars (carbohydrates) like fructose, glucose, and sucrose, they cook and behave like sugars without the caloric significance of sucrose. Psicose has 70% the sweetness of sucrose while tagatose and sorbose are nearly as sweet as table sugar. These are natural sugars that are fully absorbable by the body, but they are very low in calorie and have minimal impacts on blood sugar levels. They are also excellent humectants and as such prevent the edible from drying out. These monosaccharides have been proven to have strong antihyperglycemic and antihyperlipidemic effects, and could be used as a replacement of table sugar for the obese and diabetic subjects. The chemical structures for psicose, sorbose, and tagatose are shown in FIGURE 1.

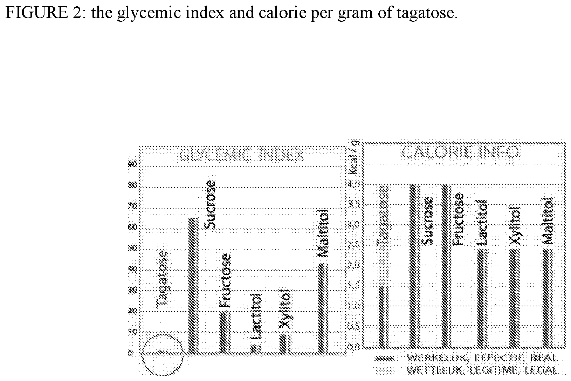

Psicose, sorbose, and tagatose are much lower in GI than glucose or sucrose levels. The low GI and low-calorie count is shown in FIGURE 2. The properties of psicose, sorbose and tagatose make them suitable for type I and II diabetics while also promoting reduction in obesity.

### Disaccharides

Trehalose, also known as mycose or tremalose, is a natural alpha-linked disaccharide formed by an α,α-1,1-glucoside bond between two α-glucose units. The carbohydrate is thought to form a gel phase as cells dehydrate, which prevents disruption of internal cell organelles, by effectively splinting them in position. Rehydration then allows normal cellular activity to be resumed without the major, lethal damage that would normally follow a dehydration/rehydration cycle. Trehalose is a non-reducing sugar and does not contain nucleophilic groups in its molecule. However, trehalose is reported to have antioxidant effects. Trehalose is also reported to have many significant neurological benefits.

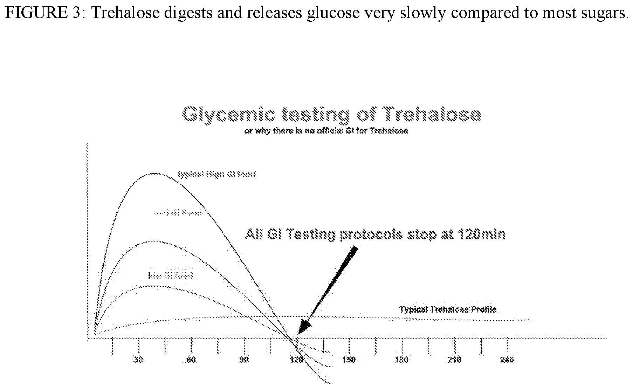

Trehalose is digested in the small intestine by the enzyme trehalase to release two molecules of glucose. However, the digestion of trehalose does not lead to a spike in glucose levels in the blood (Figure 3). Rather, the glucose levels slowly rise and are sustained over a longer period of time. Furthermore, there is no depletion of glucose over the long term, which is unlike high GI sugars. This is due to the digestion of trehalose in the small intestine rather than the upper digestive track. In addition, trehalose has a much lower instance of causing dental decay than most carbohydrates, which is another benefit of the technology.

As shown in FIGURE 3, trehalose does not cause a spike in glucose levels. Rather, trehalose leads to a long-sustained release of glucose at a low level. The sustained release of glucose prevents wild swings in insulin levels that other sugars cause. While the caloric density of trehalose is 4 Cal/g like other sugars, the lack of blood sugar level spikes makes trehalose safer for diabetics and helps prevent obesity.

### Isomaltulose (Palatinose^{®})

Isomaltulose (palatinose^{®}) is a derivative of a natural source of sucrose. Isomaltulose is made by enzymatic rearrangement of the alpha-1, 2 bond between the glucose and the fructose molecule to an alpha-1,6 bond. Isomaltulose is digested by enzymatic action by the enzyme sucrase. However, due to the rearrangement of isomaltulose vs. sucrose, sucrase hydrolysis of isomaltulose is much slower. The products of the hydrolysis are glucose and fructose. The glucose can be directly used by the body for energy while the fructose is converted to glucose by the liver. Due to its slow and full absorption and metabolic hydrolysis, isomaltulose release glucose slowly and supplies constant and long-lasting energy to the muscles and brain. Isomaltulose is slowly metabolized by the body causing only a minor increase in blood glucose level, as it enters the blood slowly. The result being that the energy obtained from Isomaltulose remains in the body much longer and offers a constant supply over a longer period than high GI sugars such as glucose or sucrose.

Like trehalose, isomaltulose only has a minor influence on the blood glucose level and a low GI value (FIGURE 3). Isomaltulose has a low GI (about 30) and therefore helps to stabilize the insulin level in the body. The products of the hydrolysis are glucose and fructose. Glucose can then be used by the body for energy while the fructose is converted to glucose by the liver. Due to its slow and full absorption and metabolic hydrolysis, isomaltulose release glucose slowly and supplies constant and long-lasting energy to the muscles and brain. Isomaltulose is slowly metabolized by the body causing only a minor increase in blood glucose level, as it enters the blood slowly. The result being that the energy obtained from Isomaltulose remains in the body much longer and offers a constant supply over a longer period than high GI sugars such as glucose or sucrose. Isomaltulose also has lower instances of tooth decay.

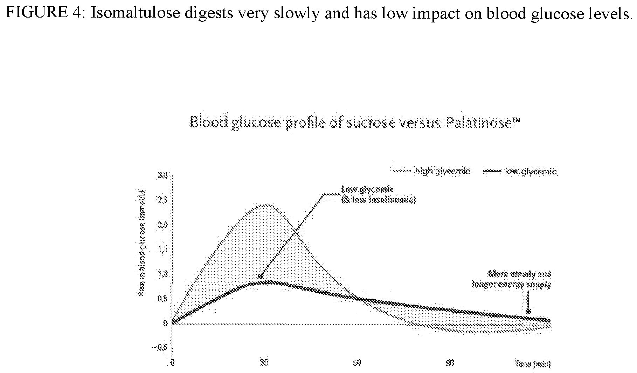

FIGURE 4 shows that isomaltulose does not cause a spike in glucose levels. Rather, isomaltulose leads to a long-sustained release of glucose at a low level. The sustained release of glucose prevents wild swings in insulin levels that other sugars cause. While the caloric density of isomaltulose is 4 Cal/g like other sugars, the lack of blood sugar level spikes makes isomaltulose safer for diabetics and helps prevent obesity.

During experimentation with the low GI compositions, it was unexpectedly discovered that mixtures of trehalose and isomaltulose seem to be sweeter than the individual components through a group panel testing. The combination of the two sugars seems to have a synergistically enhanced sweetness taste.

### N-Acetyl glucosamine

The composition may further include N-acetyl glucosamine, a naturally occurring carbohydrate. Polymers of N-acetyl glucosamine constitute Chitin, one of the most common polymers and polysaccharides. N-acetyl glucosamine also is part of hyaluronic acid, which serves as the body's natural defense, wound healing and anti-inflammation. N-acetyl glucosamine is a naturally sweet carbohydrate in its monomeric form. N-acetyl glucosamine is essential for optimal health and function in the body. It aids in cell communication. It also plays a role in how the immune system reacts with HIV and tumors. This also plays a role in osteoarthritis and helps in cartilage formation. It has been shown to play a role in nerve functioning for learning in mammals. This molecule has multiple uses such as limiting cholesterol absorption and decreases insulin secretion. Some receptors have been found in the thyroid to transport iodine proteins. It is found in multiple glands of the body and plays some role in the organ's function.

### Vitamins

The composition may further include a vitamin composition. The vitamin composition may include any vitamin or vitamin derivatives. In some embodiments, the composition includes a vitamin B composition. In some embodiments, the Vitamin B composition consists of folate, B6, and B12. In some embodiments, the vitamin B composition consists of thiamine (B1), riboflavin (B2), niacin (B3), panotothenic acid (B5), pyridoxine (B6), biotin (B7), folic acid (B9), cobalamins (B12), and derivatives or combinations thereof. In some embodiments, per daily dosing, the composition comprises at least 300mcg, 400mcg, 450mcg, or 500mcg of folate, at least 3mg, 4.5mg, 5.5mg, 6.5mg of B1, at least 3mg, 5mg, 6mg, or 7mg of B2, at least 12mg, 15mg, 16mg, 18mg, or 20mg of niacinamide, at least 4mg, 6mg, 7mg, 8mg, 10mg of B6, at least 15mcg, 20mcg, 30mcg, 50mcg, 1mg, 2mg, 4mg, 5mg, or 6mg of B12, at least 30mcg, 40mcg, 50mcg, 80mcg, or 1mg of biotin, or at least 7mg, 10mg, 12mg, 15mg, or 20mg of pantothenic acid.

In some embodiments, vitamins can include fat-soluble vitamins such as vitamin A, vitamin D, vitamin E, and vitamin K and combinations thereof. In some embodiments, vitamins can include water-soluble vitamins such as vitamin C (ascorbic acid), the B vitamins, and choline.

### Minerals

The composition may further include a mineral composition. The mineral composition may include ions of sodium, magnesium, chromium, iodine, iron, manganese, calcium, copper, fluoride, potassium, phosphorous, molybdenum, selenium, zinc, and combinations thereof. The minerals may be in the forms of salts or chelates. For sports application or medical applications, the mineral composition provided desired electrolytes.

In some embodiments, per dosage, the composition includes from at least 800mg, 1000mg, 1200mg, or 1500mg of calcium. In some embodiment, per daily dosage, the composition includes at least 10mg, 15mg, 20mg, or 30mg of iron.

### Antioxidants

The composition may further include an antioxidant composition. In some embodiments, the antioxidant composition can include but are not limited to ascorbic acid, citric acid, rosemary oil, vitamin A, vitamin E, vitamin E phosphate, tocopherols, di-alpha-tocopheryl phosphate, tocotrienols, coenzyme Q10, alpha lipoic acid, dihydrolipoic acid, xanthophylls, beta cryptoxanthin, lycopene, lutein, zeaxanthin, astaxanthin, beta-carotene, carotenes, mixed carotenoids, polyphenols, flavonoids, and combinations thereof. In some embodiments, the antioxidant composition may include extracts and active phytochemicals such as ferulic acid (from apples), ginseng, ginko biloba, beta carotene, capsicanoids, anthocyanidins, bioflavinoids, d-limonene, isothiocyanates, cysteines from garlic, ginger, grapes, catechins and polyphenols from teas, onions, phytosterols, isoflavones, lycopene, curcumin, caffeine, glucosamine, chondroitin, msm, melatonin, serotonin, and mixtures thereof.

In some embodiments, the antioxidant composition consists essentially of vitamin E, beta-carotene, and vitamin C.

### Herbal composition

The composition may include an herbal composition. The herbal composition may include herbal extracts, isolates or active phytochemicals. Examples herbal extracts and isolates include without limitation ginseng, ginko biloba, echinacea, spirulina, garlic, ginger, Leucojum aestivum, Ganoderma Lucidum, grapes, thermadrene, ma-huang, guarana, caffeine, purple willow bark, cayenne pepper, Kava kava (*kava*), St. Johns wort, Gamma oryzanol and *Tribulus terrestris, Cordyceps sinensis, Rhodiola rosea,* and *Cytoseira canariensis.* Example phytochemicals may include ferulic acid beta carotene, capsicanoids, anthocyanidins, bioflavinoids, d-limonene, isothiocyanates, cysteines, catechins and polyphenols, onions, phytosterols, isoflavones, lycopene, curcumin, caffeine, inositol hexanicotinate, glucosamine, chondroitin, msm, melatonin, serotonin, cartotenoids, chlorophyll, chlorophyllin, fiber, flavanoids, anthocyanins, cyaniding, delphinidin, malvidin, pelargonidin, peonidin, petunidin, flavanols, catechin, epicatechin, epigallocatechin, epigallocatechingallate (EGCG), theaflavins, thearubigins, proanthocyanins, flavonols, quercetin, kaempferol, myricetin, isorhamnetin, flavononeshesperetin, naringenin, eriodictyol, tangeretin, flavones, apigenin, luteolin, lignans, phytoestrogens, resveratrol, isoflavones, daidzein, genistein, glycitein, soy isoflavones, or combinations thereof.

In some embodiments, the composition may include ginseng extract or powder. Ginseng consists of several species belonging to the plant family Araliaceae. The two major forms are Chinese, Korean or Asian ginseng that belong to the genus *Panax,* and Siberian or Russian ginseng which belongs to the genus *Eleutherococcus.* The biologically active constituents in *Panax ginseng* are a complex mixture of triterpene saponins known as ginsenosides. Siberian, or Russian, ginseng consists of the dried roots and rhizome of *Eleutherococcus senticosis,* and contains phenolics, polysaccharides, and eleutherosides. In China, *Eleutherococcus senticosus* is known as *wujiaseng* or *Ciwujia* with active ingredients as ciwujianosides.

In some embodiments, the composition may include astragalus extract or powder. Astragalus extract may be water or alcohol extract of the raw herb.

In some embodiments, the herbal composition comprises ginseng, schisandra, dang-gui, he-shou-wu, gotu kola *(centella asiatica),* ashwagandha, *tribulus terrestris, mucunna pruriens,* ciwujia, or extracts or isolates thereof. In some embodiments, the herbal composition comprises ginseng or its extract or isolate thereof. In some embodiments, the ginseng comprises Asian ginseng (panax ginseng), Siberian ginseng (Eleutherococcus senticosus), American ginseng (Panax quinquefolius), indian ginseng (Withania somnifera), or a combination thereof. In some embodiments, the herbal composition comprises essentially ginseng, or its extract or isolate thereof. In some embodiments, the herbal composition comprises at least 100mg, 200mg, 300mg, 500mg, 1g of ginseng extract or isolate thereof.

In some embodiments, the herbal composition consists of ginseng and Ganoderma Lucidum. In some embodiments, the herbal composition consists of ginseng and dang-gui, or its extract or isolate thereof. In some embodiments, the herbal composition consists of ginseng and ciwujia, or its extract or isolate thereof. In some embodiments, the herbal composition consists of ginseng and ma-huang, or its extract or isolate thereof.

### Nutritional composition

The composition may further include a nutritional composition. In one embodiment, the nutritional composition comprises proteins. The protein may be milk protein, soy protein, nut protein (such almond), grain protein (such as rice protein), or bean protein. In one embodiment, the nutritional composition may include gelatin or collagen.

The composition may further include fibers and prebiotics. In one embodiment, the fibers may present in an amount of from about 0.001% to 80%, alternatively 0.001% to 5%, alternatively 5% to 10%, alternatively 10% to 15%, alternatively 15% to 40%, alternatively 40% to 60%, alternatively 60% to 80%, by weight of said composition. Any suitable fiber can be used. In one embodiment, a naturally derived fiber is used, including one or more selected from naturally derived inulin, inulin extract, synthetic inulin, hydrolysis products of inulin commonly known as fructooligosaccharides, galacto-oligosaccharides, xylooligosaccharides, oligo derivatives of starch, husks, brans, psyllium, polysaccharides, starches, polycarbophil, lignin, arabinogalactans, chitosans, oat fiber, soluble corn fiber, non-digestible corn dextrin, non-digestible wheat dextrin, locust bean gum and derivatives of locust bean gum, hydroxypropylmethyl cellulose (HPMC), pectin, and mixtures thereof.

In some embodiments, fibers may include inulin, wheat dextrin, or fructooligiosaccharides. Inulin, wheat dextrin, and fructooligiosaccharides may also act as a thickening agent and improve the texture of the composition. Various load rates of dietary fiber can be incorporated in the composition to promote a healthy digestion system, controlling blood sugar levels, and providing probiotic benefits.

Inulin is indigestible by human enzymes ptyalin and amylase, which are designed to digest starch. As a result, inulin passes through much of the digestive system intact. Inulin is a highly effective prebiotic, stimulating the growth of beneficial probiotic bacteria in the gut. Inulin is used in low fat products because of its ability to provide a creamy smooth texture to products. Inulin is a dietary fiber and is believed to activate beneficial good bacteria in the digestive tract. The activation of these bacteria is thought to reduce the risk of bowel cancer. Inulin has a mildly sweet taste, but does not affect blood sugar levels and is recommended for diabetics. Inulin has been clinically proven to increase calcium absorption. The inherent calcium in dairy foods is now an even better source of this bone-building mineral when inulin is added because inulin improves the body's uptake. People have used plants containing inulin to help relieve diabetes mellitus, a condition characterized by hyperglycemia and/or hyperinsulinemia.

In some embodiments, the nutritional composition may include probiotics. Example probiotics include, but not limited to, lactic acid producing microorganisms such as Bacillus coagulans, Bacillus subtilis, Bacillus laterosporus, Bacillus laevolacticus, Sporolactobacillus inulinus, Lactobacillus acidophilus, Lactobacillus curvatus, Lactobacillus plantarum, Lactobacillus jenseni, Lactobacillus casei, Lactobacillus fermentum, Lactococcus lactis, Pedioccocus acidilacti, Pedioccocus pentosaceus, Pedioccocus urinae, Leuconostoc mesenteroides, Bacillus coagulans, Bacillus subtilis, Bacillus laterosporus, Bacillus laevolacticus, Sporolactobacillus inulinus and mixtures thereof. Breath fresheners are also known by the following trade names:
Retsyn^{™},. Actizol^{™}, and Nutrazin^{™}. Examples of malodor-controlling compositions are also included in U.S. Pat. No. 5,300,305 to Stapler et al. and in U.S. Patent Application Publication Nos. 2003/0215417 and 2004/0081713 .

### Sweetener

Limited quantity of sweetener may be utilized as desired. In some embodiments, the amount of sweetener may be present in amounts from about 0.001% to about 1%, by weight of the composition, depending upon the sweetener or combination of sweeteners used. The exact range of amounts for each type of sweetener may be selected by those skilled in the art.

Sweeteners may include one or more monosaccharides or disaccharides. Examples include invert sugar, dextrose, lactose, honey, malt syrup, malt syrup solids, maltose, granular fructose, maple syrup, rice syrup, rice syrup solids, sorghum syrup, refiners syrup, corn syrup, corn syrup solids, high fructose corn syrup, molasses, or combinations thereof.

In one embodiment, the sweetener include common sugars, polyols such as maltitol, erythritol, and isomalt, syrup sweeteners such as glucose syrup, corn syrup, high fructose corn syrup, and juice concentrates.

In one embodiment, artificial sweeteners can be used such as acesulfame K, aspartame, sucralose, d-tagatose, neotame, monatin, and acesulfame potassium (Ace-K), or combinations thereof.

The sweeteners involved may be selected from a wide range of materials including water-soluble sweeteners, water-soluble artificial sweeteners, water-soluble sweeteners derived from naturally occurring water-soluble sweeteners, dipeptide based sweeteners, and protein based sweeteners, including mixtures thereof. Without being limited to particular sweeteners, representative categories and examples include:
(a) water-soluble sweetening agents such as dihydrochalcones, monellin, steviosides, thaumatine, lo han quo (monk fruit), lo han quo (monk fruit) derivatives, glycyrrhizin, dihydroflavenol, and sugar alcohols such as sorbitol, mannitol, maltitol, xylitol, erythritol, and L-aminodicarboxylic acid aminoalkenoic acid ester amides, such as those disclosed in U.S. Pat. No. 4,619,834, and mixtures thereof;
(b) water-soluble artificial sweeteners such as soluble saccharin salts, i.e., sodium or calcium saccharin salts, cyclamate salts, the sodium, ammonium or calcium salt of 3,4-dihydro-6-methyl-1,2,3-oxathiazine-4-one-2,2-dioxide, the potassium salt of 3,4-dihydro-6-methyl-1,2,3-oxathiazine-4-one-2,2-dioxide (Acesulfame-K), the free acid form of saccharin, and mixtures thereof;
(c) dipeptide based sweeteners, such as L-aspartic acid derived sweeteners, such as L-aspartyl-L-phenylalanine methyl ester (Aspartame), N--[N-(3,3-dimethylbutyl)-L-.alpha.-aspartyl]-L-phenylalanine 1-methyl ester (Neotame), and materials described in U.S. Pat. No. 3,492,131, L-alphaaspartyl-N-(2,2,4,4-tetramethyl-3-thietanyl)-D-alaninamide hydrate (Alitame), methyl esters of L-aspartyl-L-phenylglycerine and L-aspartyl-L-2,5-dihydrophenyl-glycine, L-aspartyl-2,5-dihydro-L-phenylalanine; L-aspartyl-L-(1-cyclohexen)-alanine, and mixtures thereof;
(d) water-soluble sweeteners derived from naturally occurring water-soluble sweeteners, such as chlorinated derivatives of ordinary sugar (sucrose), e.g., chlorodeoxysugar derivatives such as derivatives of chlorodeoxysucrose or chlorodeoxygalactosucrose, known, for example, under the product designation of Sucralose; examples of chlorodeoxysucrose and chlorodeoxygalactosucrose derivatives include but are not limited to: 1-chloro-1'-deoxysucrose; 4-chloro-4-deoxy-alpha-D-galactopyranosyl-alpha-D-fructofuranoside, or 4-chloro-4-deoxygalactosucrose; 4-chloro-4-deoxy-alpha-D-galactopyranosyl-1-chloro-1-deoxy-beta-D-fructo-- furanoside, or 4,1'-dichloro-4,1'-dideoxygalactosucrose; 1',6'-dichloro 1',6'-dideoxysucrose; 4-chloro-4-deoxy-alpha-D-galactopyranosyl-1,6-dichloro-1,6-dideoxy-beta-D- - fructofuranoside, or 4,1',6'-trichloro-4,1',6'-trideoxygalactosucrose; 4,6-dichloro-4,6-dideoxy-alpha-D-galactopyranosyl-6-chloro-6-deoxy-beta-D- -fructofuranoside, or 4,6,6'-trichloro-4,6,6'-trideoxygalactosucrose; 6,1',6'-trichloro-6,1',6'-trideoxysucrose; 4,6-dichloro-4,6-dideoxy-alpha-D-galacto-pyranosyl-1,6-dichloro-1,6-dideo- xy-beta-D-fructofuranoside, or 4,6,1',6'-tetrachloro-4,6, 1',6'-tetradeoxygalacto-sucrose; and 4,6,1',6'-tetradeoxy-sucrose, and mixtures thereof;
(e) protein based sweeteners such as thaumaoccous danielli (Thaumatin I and II) and talin; and
(f) the sweetener monatin (2-hydroxy-2-(indol-3-ylmethyl)-4-aminoglutaric acid) and its derivatives.

The intense sweetening agents may be used in many distinct physical forms well known in the art to provide an initial burst of sweetness and/or a prolonged sensation of sweetness. Without being limited thereto, such physical forms include free forms, spray dried forms, powdered forms, beaded forms, encapsulated forms, and mixtures thereof. In one embodiment, the sweetener is a high intensity sweetener such as aspartame, sucralose, and acesulfame potassium (e.g., Ace-K or acesulfame-K).

In some embodiments, the sweetener may be a polyol. Polyols can include, but are not limited to glycerol, sorbitol, maltitol, maltitol syrup, mannitol, isomalt, erythritol, xylitol, hydrogenated starch hydrolysates, polyglycitol syrups, polyglycitol powders, lactitol, and combinations thereof.

In general, an effective amount of intense sweetener may be utilized to provide the level of sweetness desired, and this amount may vary with the sweetener selected. The intense sweetener may be present in amounts from about 0.001% to about 3%, by weight of the composition, depending upon the sweetener or combination of sweeteners used. The exact range of amounts for each type of sweetener may be selected by those skilled in the art.

### Food acids

Suitable food acids may be used, including without limitation to acetic acid, adipic acid, ascorbic acid, butyric acid, citric acid, formic acid, fumaric acid, glycolic acid, lactic acid, phosphoric acid, malic acid, oxalic acid, succinic acid, tartaric acid, or combinations thereof.

Suitable buffers include but are not limited to sodium citrate and potassium citrate. For example, an acid/buffer system is 1.33% of a 54% citric acid solution, buffered with sodium citrate.

The amount of acid will be in the typical range of from about 0.5 to about 2% by weight, e.g., about 1.25%. Higher acid (lower pH) results in a lack of structure while lower acid levels do not provide enough "acid bite" in the flavor profile.

### Flavoring agents

In some embodiments, the composition may further include a flavoring agent. Flavoring agents may include those flavors known to the skilled artisan, such as natural and artificial flavors. These flavorings may be chosen from synthetic flavor oils and flavoring aromatics and/or oils, oleoresins and extracts derived from plants, leaves, flowers, fruits, and so forth, and combinations thereof.

In some embodiments, the flavoring agents may include mint(s), menthol, menthone, isomenthone, camphor and eucalyptol, eucalyptol, camphor, borneol, fenchone, menthone and isomenthone, isopulegol, monomenthyl succinate, and menthyl lactate, menthone, isomenthone, borneol, fenchone, eucalyptus, ducalyptol, ethyl benzoate, neomenthol, d-fenchone, furfurylidene butyrate, bucchu fractions, sage oil, corn mint oil, rosemary, monomenthyl succinate, amyl salicylate, eugenol, phellendrene, propyl furoate, ethyl-3-hydroxy butyrate, hexyl valerate, anisyl propionate, anysyl butyrate, dihydrocarveol, or clary sag,

Nonlimiting representative flavor oils include spearmint oil, cinnamon oil, oil of wintergreen (methyl salicylate), peppermint oil, Japanese mint oil, clove oil, bay oil, anise oil, eucalyptus oil, thyme oil, cedar leaf oil, oil of nutmeg, allspice, oil of sage, mace, oil of bitter almonds, and cassia oil. Also useful flavorings are artificial, natural and synthetic fruit flavors such as vanilla, and citrus oils including lemon, orange, lime, grapefruit, yazu, sudachi, and fruit essences including apple, pear, peach, grape, blueberry, strawberry, raspberry, cherry, plum, pineapple, apricot, banana, melon, apricot, ume, cherry, raspberry, blackberry, tropical fruit, mango, mangosteen, pomegranate, papaya and so forth. Other potential flavors whose release profiles can be managed include a milk flavor, a butter flavor, a cheese flavor, a cream flavor, and a yogurt flavor; a vanilla flavor; tea or coffee flavors, such as a green tea flavor, a oolong tea flavor, a tea flavor, a cocoa flavor, a chocolate flavor, and a coffee flavor; mint flavors, such as a peppermint flavor, a spearmint flavor, and a Japanese mint flavor; spicy flavors, such as an asafetida flavor, an ajowan flavor, an anise flavor, an angelica flavor, a fennel flavor, an allspice flavor, a cinnamon flavor, a camomile flavor, a mustard flavor, a cardamom flavor, a caraway flavor, a cumin flavor, a clove flavor, a pepper flavor, a coriander flavor, a sassafras flavor, a savory flavor, a Zanthoxyli Fructus flavor, a perilla flavor, a juniper berry flavor, a ginger flavor, a star anise flavor, a horseradish flavor, a thyme flavor, a tarragon flavor, a dill flavor, a capsicum flavor, a nutmeg flavor, a basil flavor, a marjoram flavor, a rosemary flavor, a bayleaf flavor, and a wasabi (Japanese horseradish) flavor; alcoholic flavors, such as a wine flavor, a whisky flavor, a brandy flavor, a rum flavor, a gin flavor, and a liqueur flavor; floral flavors; and vegetable flavors, such as an onion flavor, a garlic flavor, a cabbage flavor, a carrot flavor, a celery flavor, mushroom flavor, and a tomato flavor. These flavoring agents may be used in liquid or solid form and may be used individually or in admixture. Commonly used flavors include mints such as peppermint, menthol, spearmint, artificial vanilla, cinnamon derivatives, and various fruit flavors, whether employed individually or in admixture. Flavors may also provide breath freshening properties, particularly the mint flavors when used in combination with the cooling agents, described herein below. In some embodiments, flavorants may chose from geraniol, linalool, nerol, nerolidal, citronellol, heliotropine, methyl cyclopentelone, ethyl vanillin, maltol, ethyl maltol, furaneol, alliaceous compounds, rose type compounds such as phenethanol, phenylacetic acid, nerol, linalyl esters, jasmine, sandlewood, patchouli, and/or cedarwood.

In some embodiments, other flavorings include aldehydes and esters such as cinnamyl acetate, cinnamaldehyde, citral diethylacetal, dihydrocarvyl acetate, eugenyl formate, p-methylamisol, and so forth may be used. Generally any flavoring or food additive such as those described in Chemicals Used in Food Processing, publication 1274, pages 63-258, by the National Academy of Sciences, may be used. These may include natural as well as synthetic flavors.

Further examples of aldehyde flavorings include but are not limited to acetaldehyde (apple), benzaldehyde (cherry, almond), anisic aldehyde (licorice, anise), cinnamic aldehyde (cinnamon), citral, i.e., alpha-citral (lemon, lime), neral, i.e., beta-citral (lemon, lime), decanal (orange, lemon), ethyl vanillin (vanilla, cream), heliotrope, i.e., piperonal (vanilla, cream), vanillin (vanilla, cream), alpha-amyl cinnamaldehyde (spicy fruity flavors), butyraldehyde (butter, cheese), valeraldehyde (butter, cheese), citronellal (modifies, many types), decanal (citrus fruits), aldehyde C-8 (citrus fruits), aldehyde C-9 (citrus fruits), aldehyde C-12 (citrus fruits), 2-ethyl butyraldehyde (berry fruits), hexenal, i.e., trans-2 (berry fruits), tolyl aldehyde (cherry, almond), veratraldehyde (vanilla), 2,6-dimethyl-5-heptenal, e.g., melonal (melon), 2,6-dimethyloctanal (green fruit), and 2-dodecenal (citrus, mandarin), cherry, grape, blueberry, blackberry, strawberry shortcake, and mixtures thereof.

In some embodiments, flavoring agents are used at levels that provide a perceptible sensory experience i.e. at or above their threshold levels. In other embodiments, flavoring agents are used at levels below their threshold levels such that they do not provide an independent perceptible sensory experience. At subthreshold levels, the flavoring agents may provide an ancillary benefit such as flavor enhancement or potentiation.

In some embodiments, a flavoring agent may be employed in either liquid form and/or dried form. When employed in the latter form, suitable drying means such as spray drying the liquid may be used. Alternatively, the flavoring agent may be absorbed onto water-soluble materials, such as cellulose, starch, sugar, maltodextrin, gum arabic and so forth or may be encapsulated. In still other embodiments, the flavoring agent may be adsorbed onto silicas, zeolites, and the like.

In some embodiments, the flavoring agents may be used in many distinct physical forms. Without being limited thereto, such physical forms include free forms, such as spray dried, powdered, beaded forms, encapsulated forms, and mixtures thereof.

### Sensate agents

In some embodiment, the composition further includes a sensate agent. Sensate agents can include cooling agents, warming agents, tingling agents, effervescent agents, and combinations thereof. A variety of cooling agents may be employed. For example, among the useful cooling agents are included xylitol, erythritol, dextrose, sorbitol, menthane, menthone, ketals, menthone ketals, menthone glycerol ketals, substituted p-menthanes, acyclic carboxamides, mono menthyl glutarate, substituted cyclohexanamides, substituted cyclohexane carboxamides, substituted ureas and sulfonamides, substituted menthanols, hydroxymethyl and hydroxymethyl derivatives of p-menthane, 2-mercapto-cyclo-decanone, hydroxycarboxylic acids with 2-6 carbon atoms, cyclohexanamides, menthyl acetate, menthyl salicylate, N,2,3-trimethyl-2-isopropyl butanamide (WS-23), N-ethyl-p-menthane-3-carboxamide (WS-3), isopulegol, 3-(1-menthoxy)propane-1,2-diol, 3-(1-menthoxy)-2-methylpropane-1,2-diol, p-menthane-2,3-diol, p-menthane-3,8-diol, 6-isopropyl-9-methyl-1,4-dioxaspiro[4,5]decane-2-methanol, menthyl succinate and its alkaline earth metal salts, trimethylcyclohexanol, N-ethyl-2-isopropyl-5-methylcyclohexanecarboxamide, Japanese mint oil, peppermint oil, 3-(1-menthoxy)ethan-1-ol, 3-(1-menthoxy)propan-1-ol, 3-(1-menthoxy)butan-1-ol, 1-menthylacetic acid N-ethylamide, 1-menthyl-4-hydroxypentanoate, 1-menthyl-3-hydroxybutyrate, N,2,3-trimethyl-2-(1-methylethyl)-butanamide, n-ethyl-t-2-c-6 nonadienamide, N,N-dimethyl menthyl succinamide, substituted p-menthanes, substituted p-menthane-carboxamides, 2-isopropanyl-5-methylcyclohexanol (from Hisamitsu Pharmaceuticals, hereinafter "isopregol"); menthone glycerol ketals (FEMA 3807, tradename FRESCOLAT.RTM. type MGA); 3-1-menthoxypropane-1,2-diol (from Takasago, FEMA 3784); and menthyl lactate; (from Haarman & Reimer, FEMA 3748, tradename FRESCOLAT.RTM. type ML), WS-30, WS-14, Eucalyptus extract (p-Mehtha-3,8-Diol), Menthol (its natural or synthetic derivatives), Menthol PG carbonate, Menthol EG carbonate, Menthol glyceryl ether, N-tertbutyl-p-menthane-3-carboxamide, P-menthane-3-carboxylic acid glycerol ester, Methyl-2-isopryl-bicyclo (2.2.1), Heptane-2-carboxamide; and Menthol methyl ether, and menthyl pyrrolidone carboxylate among others. These and other suitable cooling agents are further described in the following U.S. patents: U.S. Pat. Nos. 4,230,688; 4,032,661; 4,459,425; 4,136,163; 5,266,592; 6,627,233.

In some embodiments, warming components may be selected from a wide variety of compounds known to provide the sensory signal of warming to the user. These compounds offer the perceived sensation of warmth, particularly in the oral cavity, and often enhance the perception of flavors, sweeteners and other organoleptic components. In some embodiments, useful warming compounds can include vanillyl alcohol n-butylether (TK-1000) supplied by Takasago Perfumary Company Limited, Tokyo, Japan, vanillyl alcohol n-propylether, vanillyl alcohol isopropylether, vanillyl alcohol isobutylether, vanillyl alcohol n-aminoether, vanillyl alcohol isoamyleather, vanillyl alcohol n-hexyleather, vanillyl alcohol methylether, vanillyl alcohol ethylether, gingerol, shogaol, paradol, zingerone, capsaicin, dihydrocapsaicin, nordihydrocapsaicin, homocapsaicin, homodihydrocapsaicin, ethanol, isopropyl alcohol, iso-amylalcohol, benzyl alcohol, glycerine, and combinations thereof.

In some embodiments, a tingling sensation can be provided. One such tingling sensation is provided by adding jambu, oleoresin, or spilanthol to some examples. In some embodiments, alkylamides extracted from materials such as jambu or sanshool can be included. Additionally, in some embodiments, a sensation is created due to effervescence. Such effervescence is created by combining an alkaline material with an acidic material. In some embodiments, an alkaline material can include alkali metal carbonates, alkali metal bicarbonates, alkaline earth metal carbonates, alkaline earth metal bicarbonates and mixtures thereof. In some embodiments, an acidic material can include acetic acid, adipic acid, ascorbic acid, butyric acid, citric acid, formic acid, fumaric acid, glyconic acid, lactic acid, phosphoric acid, malic acid, oxalic acid, succinic acid, tartaric acid and combinations thereof. Examples of "tingling" type sensates can be found in U.S. Pat. No. 6,780,443.

Sensate agents may also be referred to as "trigeminal stimulants" such as those disclosed in U.S. Patent Application No. 205/0202118. Trigeminal stimulants are defined as an orally consumed product or agent that stimulates the trigeminal nerve. Examples of cooling agents which are trigeminal stimulants include menthol, WS-3, N-substituted p-menthane carboxamide, acyclic carboxamides including WS-23, methyl succinate, menthone glycerol ketals, bulk sweeteners such as xylitol, erythritol, dextrose, and sorbitol, and combinations thereof. Trigeminal stimulants can also include flavors, tingling agents, Jambu extract, vanillyl alkyl ethers, such as vanillyl n-butyl ether, spilanthol, Echinacea extract, Northern Prickly Ash extract, capsaicin, capsicum oleoresin, red pepper oleoresin, black pepper oleoresin, piperine, ginger oleoresin, gingerol, shoagol, cinnamon oleoresin, cassia oleoresin, cinnamic aldehyde, eugenol, cyclic acetal of vanillin and menthol glycerin ether, unsaturated amides, and combinations thereof.

In some embodiments, sensate agents are used at levels that provide a perceptible sensory experience i.e. at or above their threshold levels. In other embodiments, sensate components are used at levels below their threshold levels such that they do not provide an independent perceptible sensory experience. At subthreshold levels, the sensates may provide an ancillary benefit such as flavor or sweetness enhancement or potentiation.

### Freshening agents

In some embodiments, the composition further includes a freshening agent. Freshening agents may include essential oils as well as various aldehydes, alcohols, and similar materials. In some embodiments, essential oils can include oils of spearmint, peppermint, wintergreen, sassafras, chlorophyll, citral, geraniol, cardamom, clove, sage, carvacrol, eucalyptus, cardamom, magnolia bark extract, marjoram, cinnamon, lemon, lime, grapefruit, and orange. In some embodiments, aldehydes such as cinnamic aldehyde and salicylaldehyde can be used. Additionally, chemicals such as menthol, carvone, iso-garrigol, and anethole can function as breath fresheners. Of these, the most commonly employed are oils of peppermint, spearmint and chlorophyll.

In addition to essential oils and chemicals derived from them, in some embodiments freshening agent can include but are not limited to zinc citrate, zinc acetate, zinc fluoride, zinc ammonium sulfate, zinc bromide, zinc iodide, zinc chloride, zinc nitrate, zinc fluorosilicate, zinc gluconate, zinc tartarate, zinc succinate, zinc formate, zinc chromate, zinc phenol sulfonate, zinc dithionate, zinc sulfate, silver nitrate, zinc salicylate, zinc glycerophosphate, copper nitrate, chlorophyll, copper chlorophyll, chlorophyllin, hydrogenated cottonseed oil, chlorine dioxide, beta cyclodextrin, zeolite, silica-based materials, carbon-based materials, enzymes such as laccase, and combinations thereof.

### Coloring agents

Coloring agents may be used in amounts effective to produce the desired color. The coloring agents may include pigments, which may be incorporated in amounts up to about 6%, by weight of the composition. For example, titanium dioxide may be incorporated in amounts up to about 2%, and preferably less than about 1%, by weight of the composition. The colorants may also include natural food colors and dyes suitable for food, drug and cosmetic applications. These colorants are known as F.D.& C. dyes and lakes. The materials acceptable for the foregoing uses are preferably water-soluble. Illustrative nonlimiting examples include the indigoid dye known as F.D.& C. Blue No. 2, which is the disodium salt of 5,5-indigotindisulfonic acid. Similarly, the dye known as F.D.& C. Green No. 1 comprises a triphenylmethane dye and is the monosodium salt of 4-[4-(N-ethyl-p-sulfoniumbenzylamino) diphenylmethylene]-[1-(N-ethyl-N-p-sulfoniumbenzyl)-delta-2,5-cyclohexadi- eneimine]. A full recitation of all F.D.& C. colorants and their corresponding chemical structures may be found in the Kirk-Othmer Encyclopedia of Chemical Technology, 3rd Edition, in volume 5 at pages 857-884.

In some embodiments, one or more colors can be included. As classified by the United States Food, Drug, and Cosmetic Act (21 C.F.R. 73), colors can include exempt from certification colors (sometimes referred to as natural even though they can be synthetically manufactured) and certified colors (sometimes referred to as artificial), or combinations thereof. In some embodiments, exempt from certification or natural colors can include, but are not limited to annatto extract, (E160b), bixin, norbixin, astaxanthin, dehydrated beets (beet powder), beetroot red/betanin (E162), ultramarine blue, canthaxanthin (E161g), cryptoxanthin (E161c), rubixanthin (E161d), violanxanthin (E161e), rhodoxanthin (E161f), caramel (E150(a-d)), .beta.-apo-8'-carotenal (E160e), beta.-carotene (E160a), alpha carotene, gamma carotene, ethyl ester of beta-apo-8 carotenal (E160f), flavoxanthin (E161a), lutein (E161b), cochineal extract (E120); carmine (E132), carmoisine/azorubine (E122), sodium copper chlorophyllin (E141), chlorophyll (E140), toasted partially defatted cooked cottonseed flour, ferrous gluconate, ferrous lactate, grape color extract, grape skin extract (enocianina), anthocyanins (E163), haematococcus algae meal, synthetic iron oxide, iron oxides and hydroxides (E172), fruit juice, vegetable juice, dried algae meal, tagetes (Aztec marigold) meal and extract, carrot oil, corn endosperm oil, paprika, paprika oleoresin, phaffia yeast, riboflavin (E101), saffron, titanium dioxide, turmeric (E100), turmeric oleoresin, amaranth (E123), capsanthin/capsorbin (E160c), lycopene (E160d), and combinations thereof.

In some embodiments, certified colors can include, but are not limited to, FD&C blue #1, FD&C blue #2, FD&C green #3, FD&C red #3, FD&C red #40, FD&C yellow #5 and FD&C yellow #6, tartrazine (E102), quinoline yellow (E104), sunset yellow (E110), ponceau (E124), erythrosine (E127), patent blue V (E131), titanium dioxide (E171), aluminium (E173), silver (E174), gold (E175), pigment rubine/lithol rubine BK (E180), calcium carbonate (E170), carbon black (E153), black PN/brilliant black BN (E151), green S/acid brilliant green BS (E142), and combinations thereof. In some embodiments, certified colors can include FD&C aluminum lakes. These include of the aluminum salts of FD&C dyes extended on an insoluble substrate of alumina hydrate. Additionally, in some embodiments, certified colors can be included as calcium salts.

In some embodiments, natural fruits or plant juice or extracts may be used as the coloring agents. Example include without limitation carrot juice, raspberry juice, blackberry juice, blueberry juice, and beet juice.

### Plasticizer

In some embodiments, the composition may further include plasticizer to modify the texture of the formulation. A texture-modifying agent may include a particulate material. Suitable particulate materials can include, but are not limited to, sucrose, polyols such as sorbitol, xylitol, mannitol, galactitol, lactitol, maltitol, erythritol, isomalt, hydrogenated starch hydrolysates and mixtures thereof, starches, proteins, and combinations thereof. In some embodiments, the particulate material serving as a texture modifying component is selected based on its ability or lack of ability to crystallize the saccharides in the saccharide portion. For example, when isomalt is included in the saccharide portion, sorbitol powder can be added to the composition because it will not cause the isomalt to crystallize. Alternatively, when erythritol is included in the saccharide portion, erythritol powder can be added to the composition because it will cause the erythritol to crystallize. Such particulates can be included in amounts from 5% to 35% w/w of the composition.

### Fats and oils

In some embodiments, a texture-modifying component can include fats, oils, or other hydrophobic materials. Suitable fats can include, but are not limited to, partially hydrogenated vegetable or animal fats, such as coconut oil, corn oil, palm kernel oil, peanut oil, soy bean oil, sesame oil, cottonseed oil, cocoa butter, milk fat, beef tallow, and lard, among others. Suitable hydrophobic materials include chocolate, chocolate crumb, carob coatings, and compound coatings. Such fats, oils, and/or hydrophobic materials can be included in amounts of 1% to 10% w/w of the composition.

In some embodiments, the sensory perception of the texture-modifying component is similar to that of fat, oil, or other hydrophobic materials. For example, a composition including 2.5% fats or oil can provide the same mouthfeel perception as a composition including 10%-50% fat as measured by sensory evaluation techniques.

Suitable oils and fats usable in compositions include vegetable or animal fats, such as butter, coconut oil, palm kernel oil, beef tallow, and lard, among others. These ingredients when used may be present in amounts up to about 7%, or up to about 3.5%, by weight of the composition.

In some embodiments, the composition may include edible oil component present in an amount of from about 1% to about 30%, alternatively 1% to 5%, alternatively 5% to 10%, alternatively 10% to 15%, alternatively 15% to 20%, alternatively 20% to 25%, alternatively 25% to 30%, by weight of the composition. In some embodiments, the edible oil component may be present in an amount of from about 0% to about 30%, alternatively 0% to 1%, alternatively 1% to 5%, alternatively 5% to 10%, alternatively 10% to 15%, alternatively 15% to 20%, alternatively 20% to 25%, alternatively 25% to 30%, by weight of the composition. This edible oil component makes up part of the group of ingredients that adjust the taste, texture, and improve the melt and mouth feel of the flavored chewy or edible confection. For example, in some embodiments, the interaction of the group of highly unsaturated oils with the coconut oil component may create an improved elasticity within the flavored chewy confection that acts similar to hydrogenated or partially hydrogenated fat. The edible oil component also improves the health characteristic of the flavored chewy confection compositions because it adds monounsaturated and polyunsaturated fats. An example of an edible oil component is a blend of canola, soybean oil, and sunflower oil.

Non-limiting examples of edible oil components acceptable for use in the preferred embodiments include those that have low saturated fat content and high unsaturated fat including monounsaturated and especially polyunsaturated oils. The edible oil component should have no specific flavor and preferably is basically bland or somewhat buttery in taste. The edible oils component can be selected from the following; canola oil, soybean oil, safflower oil, sunflower oil, sesame oil, walnut oil, olive oil, flaxseed oil, chia seed oil, almond oil, corn oil, grape seed oil, peanut oil, other nut oils, and synthesized or reorganized oils, and combinations thereof.

In some embodiment, the edible oil component may have a high level of saturated fats present in an amount of from about 0.3% to about 20%, alternatively 0.3% to 3%, alternatively 3% to 5%, alternatively 5% to 10%, alternatively 10% to 15%, alternatively 15% to 20%, by weight of the composition. Suitable oils having a high level of saturated fats include, but are not limited to, one or more selected from the group consisting of coconut oil, palm oil, fractionated coconut or palm oil, partially hydrogenated coconut or palm oil, fully hydrogenated coconut or palm oil, or any other synthesized or altered edible oils including partially hydrogenated oils and fully hydrogenated oils that have either highly saturated or highly unsaturated fatty acids that when hydrogenated become solid similar to coconut oil in consistency including partially hydrogenated soybean oil, cotton seed oil, palm kernel oil or combination of these edible oils. In one embodiment, the edibe oil component comprises coconut oil. This oil component forms a part of the flavor profile and provides a texture to the flavored chewy or edible confection, and it improves the taste, texture, melt, and mouth feel of the compositions. The blend of the flavor components provides for a great taste, texture, melt and mouthfeel, without the necessity of using partially hydrogenated or fully hydrogenated oils. Any medium heat processed coconut oil can be used.

### Humectant

The glycerin is a humectant and freezing point depressant. It also helps decrease the tendency for granulation and aid in maintaining softness. In some embodiment, glycerin or equivalent material may be employed at a level of from about 1 to about 5% by weight of the final product, e.g., 2 to 3%.

Humectants that can provide a perception of mouth hydration may be included. Such humectants can include, but are not limited to glycerol, sorbitol, polyethylene glycol, erythritol, and xylitol. Additionally, in some embodiments, fats can provide a perception of mouth moistening. Such fats can include medium chain triglycerides, vegetable oils, fish oils, mineral oils, and combinations thereof.

### Bulking agents

Suitable sugar bulking agents include monosaccharides, disaccharides and polysaccharides such as xylose, ribulose, glucose (dextrose), lactose, mannose, galactose, fructose (levulose), sucrose (sugar), maltose, invert sugar, partially hydrolyzed starch and corn syrup solids, and mixtures thereof.

Suitable sugar alcohol bulking agents include sorbitol, xylitol, mannitol, galactitol, lactitol, maltitol, erythritol, isomalt and mixtures thereof. Suitable hydrogenated starch hydrolysates include those disclosed in U.S. Pat. No. 4,279,931 and various hydrogenated glucose syrups and/or powders which contain sorbitol, maltitol, hydrogenated disaccharides, hydrogenated higher polysaccharides, or mixtures thereof. Hydrogenated starch hydrolysates are primarily prepared by the controlled catalytic hydrogenation of corn syrups. The resulting hydrogenated starch hydrolysates are mixtures of monomeric, dimeric, and polymeric saccharides. The ratios of these different saccharides give different hydrogenated starch hydrolysates different properties. Mixtures of hydrogenated starch hydrolysates, such as LYCASIN^{™}, a commercially available product manufactured by Roquette Freres of France, and HYSTAR.RTM., a commercially available product manufactured by SPI Polyols, Inc. of New Castle, Del., are also useful.

### Emulsifiers

The composition may include an emulsifier. The emulsifier may present in an amount of from about 0.001% to about 5%, alternatively 0.001% to 1%, alternatively 1% to 3%, alternatively 3% to 5%, by weight of the composition. In some embodiments, the emulsifier present in an amount of from about 0% to about 5%, alternatively 0.001% to 1%, alternatively 1% to 3%, alternatively 3% to 5%, by weight of the composition.

Example emulsifiers include but not limited to modified cornstarch, mono-and diglycerides, and lecithin.

The emulsifier may assist in holding together the fats and water and other components together in a homogeneous composition. In one embodiment, the emulsifier may assist in the formation of a "water and oil" emulsion that creates the smooth texture of the finished product.

### Liquids

Liquids may be used to assist in the flavoring and texture profile of the products. In some embodiments, the composition may include from about 0.001% to about 25% by weight of a fruit or vegetable or combination juice or concentrate component, alternatively 0.001% to 5%, alternatively 5% to 10%, alternatively 10% to 15%, alternatively 15% to 20%, alternatively 20% to 25%, by weight of the composition. The fruit or vegetable or combination juice or concentrate component adds a flavor to the flavored chewy or edible confection. Any suitable source from the following may used in the embodiments; citrus fruit juices, orchard fruit juices, berry fruit juices, vine fruit juices, decolorized juices, and vegetable juices can be used for this component. The forms can come from juices or concentrates of fruits or vegetables.

In some embodiments, the composition may also include a water component present in an amount of about 1% or greater. The water component adds to the overall texture and melt and chewiness of the flavored chewy or sweet composition. For example, water may be used because of the increase in viscosity of some example compositions. In some embodiments, the composition may contain water from about 1% to about 20%, alternatively 1% to 5%, alternatively 5% to 10%, alternatively 10% to 15%, alternatively 15% to 20%, by weight of the composition.

### Preservatives

Preservatives may be natural or synthetic. Non-limiting examples of suitable preservatives include: sodium benzoate, sodium citrate, sodium phosphate, potassium metabisulfite, sodium metabisulfite, sodium lactate, sodium sulfite, EDTA (ethylenediaminetetraacetic acid), methylparaben, TBHQ, tocopherols, and mixtures thereof. Natural preservatives may include phenols (phenolic acid, polyphenols, tannins), isoflavonoids, organic acids (acetic, lactic, citric), and herb extracts such as extracts of citrus fruits, oregano, thyme, sage, rosemary, clove, coriander, garlic, and onion.

In some embodiments, the composition may include at least about 0% to 2%, by weight of the composition of a preservative component from above, or mixtures thereof.

### Thickening agent

The composition may further include a thickening agent to help with the viscosity of the final product. Some thickening agents are gelling agents. Others act as mechanical thixotropic additives with discrete particles adhering or interlocking to resist strain.

In some embodiments, the thickening agent may be polysaccharides or protein. Example polysaccharides thickening agents include starches, vegetable gums and pectin. Example starch based thickening agents include arrowroot, cornstarch, katakuri starch, potato starch, sago, tapioca and their starch derivatives. Example vegetable gums based thickening agents may include alginin, guar gum, locust bean gum, and xanthan gum. Example protein based thickening agents include collagen, egg whites, furcellaran, and gelatin. Sugar based thickening agent may include agar and carrageenan.

### Methods of making

The application provides methods for making the claimed composition.

The method may include the steps of sifting together or mixing various ingredients into the desired composition. The method may include mixing or dissolving various ingredients into a solution and spray dry or freeze dry the solution. Those skilled in the art are capable of designing a suitable processing procedure to prepare the desired products.

The embodiments now will be described in more detail with reference to the non-limiting examples that follow.

### EXAMPLES

### Example 1. Low GI Syrup

Ingredients: Psicose, Water, Isomaltulose, Trehalose, Natural and Artificial Flavor, Malic Acid, Xanthan Gum, Sodium Chloride, Sodium Hexametaphosphate, Preservatives (Sodium Benzoate, Sorbic Acid), Caramel Color, Phosphoric Acid, Pectin.

Method: 1000 grams of psicose is added to a heating container followed by 495 grams isomaltulose and 495 grams trehalose. Psicose, isomaltulose, and trehalose are then blended together with 10 grams of xanthan gum until homogeneous. To the mixture is then added 700 grams water. The solution is heated to the boiling point and malic acid added. To the boiling solution is added 2 grams sodium chloride followed by 1.5 grams each of sodium hexametaphosphate, sodium benzoate and potassium sorbate. 0.85 grams of phosphoric acid is added to the solution. The system is heated until a Brix level of 74 is reached, removal from heat, and then followed by the addition of 15 grams of maple flavoring and caramel color.

### Example 2. Low GI Powder Flavoring.

Ingredients: Psicose, Water, Isomaltulose, Trehalose, Malic Acid, Natural and Artificial Flavor, FD&C Red No. 40.

Method: 1000 grams of psicose is added to a heating container followed by 495 grams isomaltulose, 495 grams trehalose and 35 grams of malic acid. The components are blended together until homogeneous and 400 grams of water is added. The system is heated to a vigorous boil and water is removed until a Brix of 93 is reached at which time 20 grams of a slurry of FD&C red No. 40 is added. The heat is removed and 90 grams of fruit punch flavoring is added. 1-gram pellets are formed from the hot mixture and allowed to cool to room temperature. The pellets are then ground to a 40 -120 mesh powder.

### Example 3. Low GI High Vitamin C Evanescence Powder with added Calcium, Magnesium, Potassium, and Zinc.

Ingredients: The low GI power from Example 2. Zinc Ascorbate, Ascorbic Acid, Calcium Citrate, Magnesium Citrate, Potassium Citrate.

Method: 19.6 grams calcium citrate, 35.3 grams magnesium citrate, 49.6 grams potassium citrate, 47.1 grams each of ascorbic acid and zinc ascorbate are blended with 801.3 grams of the low GI powder flavoring from Example 2 and made homogeneous.

### Example 4. Low GI High Vitamin C Evanescence Tablet with added Calcium, Magnesium, Potassium, and Zinc.

Ingredients: The low GI power from Example 2. Zinc Ascorbate, Ascorbic Acid, Calcium Citrate, Magnesium Citrate, Potassium Citrate.

Method: 19.6 grams calcium citrate, 35.3 grams magnesium citrate, 49.6 grams potassium citrate, 47.1 grams each of ascorbic acid and zinc ascorbate are blended with 801.3 grams of the low GI powder flavoring from Example 2 and made homogeneous.

5 grams of powder is introduced into the die cavity. The dry powder is then compacted at a pressure of 1015 psi to yield tablets. Optionally, the tablets can be coated with carboxymethylcellulose.

### Example 5. Low GI Cough Syrup

Ingredients: Psicose, Isomaltulose, Trehalose, Xanthan Gum, Malic Acid, Levocetirizine Dihydrochloride, Dextromethorphan HCl, Guaifenesin, Chlorpheniramine Maleate, Phenylephrine HCl, Sodium Benzoate, Prepared Psicose/Trehalose/Isomaltulose Syrup, Potassium Citrate, Sodium Chloride, Ethanol, SS, Glycerol, Color, Flavor, Purified Water.

Method: 1000 grams of psicose is added to a heating container followed by 495 grams isomaltulose and 495 grams trehalose. Psicose, isomaltulose, and trehalose are then blended together with 10 grams of xanthan gum until homogeneous. To the mixture is then added 700 grams water. The solution is heated to the boiling point and malic acid added. To the boiling solution is added 2 grams sodium chloride followed by 1.5 grams each of sodium hexametaphosphate, sodium benzoate and potassium sorbate. The system is heated until a Brix level of 81 is reached.

To 1000 mL water with stirring is added 50 mL glycerol, 2.0 grams dextromethorphan HCl, 2.0 grams levocetirizine dihydrochloride, 10.0 grams guaifenesin, 0.2 grams chlorpheniramine maleate, phenylephrine HCl, 5.2 grams sodium chloride, and 50 mL ethanol and the components are allowed to fully dissolve. To the solution is added 5 grams of FD&C No. 40 paste and allowed to fully disperse. To the solution is then added 500 mL of the psicose, trehalose, isomaltulose syrup created above. To the solution is then added 4.5 grams of Cherry flavor.

### Example 6. Low GI Non-dairy Creamer

Ingredients: Psicose, Isomaltulose, Trehalose, Xanthan Gum, Malic Acid, Sodium Benzoate, Potassium Sorbate, High Lauric Vegetable Fat, Sodium Caseinate, Mono/diglyceride Emulsifier (Cremodon 250/20), Dipotassium Hydrogen Phosphate, Sodium Carbonate.

Method: A emulsion is prepared, by first dissolving 27.35 kg psicose, 13.54 kg trehalose, 13.54 kg isomaltulose and 7.0 kg sodium caseinate in 90.7 kg warm water at 50° C., followed by the addition of 3 kg of the emulsifier. To this was dissolved 2.45 kg of dipotassium hydrogen phosphate and 0.27 kg of sodium carbonate. A melted fat phase was prepared from high lauric coconut oil at the same time and kept at a temperature no higher than 50° C. 31 kg of the melted fat phase was then poured into the aqueous phase with agitation to cause emulsification, at a temperature no higher than 50° C. The resultant emulsion was then homogenized under a pressure of 4000 psi. To the solution is added 0.85 kg of almond flavor and color.

Optionally, the above emulsion is passed to a spray drier of conventional design with centrifugal pressure nozzles and dried to a powder of 2% moisture content. An inlet temperature of 400° F. and an outlet temperature of 200° F. was employed, to give somewhat less than 98 kg dried product.

### Comparative Example 7. Low GI Electrolyte Solution

Ingredients: Tagatose, Isomaltulose, Sodium Citrate, Potassium Citrate.

Method: To 964.1 grams of purified water is added 14 grams of tagatose and 11 grams of isomaltulose with stirring. The components are allowed to dissolve. To this solution are added 2.053 grams potassium citrate and 3.833 grams sodium citrate and the electrolytes are allowed to dissolve. The solution was heated to 85 °C for 30 minutes followed by the addition of 4 grams orange flavor and 1 gram of orange color.

### Example 8. Low GI Candy

Ingredients: Psicose, Water, Isomaltulose, Trehalose, Malic Acid, Citric Acid, Natural and Artificial Flavor, FD&C Red No. 40.

Method: 1000 grams of psicose is added to a heating container followed by 495 grams isomaltulose, 495 grams trehalose and 15 grams of malic acid. The components are blended together until homogeneous and 400 grams of water is added. The system is heated to a vigorous boil and water is removed until a Brix of 93 is reached at which time 8 grams of a slurry of FD&C red No. 40 is added. The heat is removed and 9 grams of watermelon flavoring is added. 2.5 - 5-gram shapes are formed from the hot mixture by pouring the hot liquid mixture into a silicone mold and allowed to cool to room temperature.

### Example 9. Low GI Dark Chocolate

Ingredients: Psicose, Water, Isomaltulose, Trehalose, Malic Acid

Method: 1000 grams of psicose is added to a heating container followed by 495 grams isomaltulose, 495 grams trehalose and 3 grams of malic acid. The components are blended together until homogeneous and 400 grams of water is added. The system is heated to a vigorous boil and water is removed until a Brix of 81 is reached. The solution is cooled to 100 °C.

Gently melt 9490 grams of 92-degree coconut oil medium-low heat to 100 °C. To the heated oil is slowly added 4034 grams cocoa powder with stirring. The cocoa powder is allowed to fully incorporate. Once the mixture is homogeneous, the sweetener solution prepared above is slowly added with stirring. To the homogeneous solution is then added 131 grams of vanilla extract. Optionally, 15 grams of orange flavor or 15 grams raspberry flavor is added. Pour mixture into a candy mold or pliable tray. Refrigerate until chilled, about 1 hour.

### Example 10. Low GI Milk Chocolate

Ingredients: Psicose, Water, Isomaltulose, Trehalose, Malic Acid, Cocoa Butter, Full Fat Milk Powder, Cocoa Powder

Method: 1000 grams of psicose is added to a heating container followed by 495 grams isomaltulose, 495 grams trehalose and 3 grams of malic acid. The components are blended together until homogeneous and 400 grams of water is added. The system is heated to a vigorous boil and water is removed until a Brix of 85 is reached. The solution is cooled to 100 °C and maintained at that temperature.

Gently melt 2000 grams of cocoa butter and heat to 100 °C. To the heated cocoa butter is slowly added 1000 grams of finely powdered full fat milk powder and fully dissolve into the cocoa butter. To the heated mixture is slowly added 600 grams cocoa powder with stirring. The cocoa powder is allowed to fully incorporate. Once the mixture is homogeneous, the sweetener solution prepared above is slowly added with stirring. To the homogeneous solution is then added 131 grams of vanilla extract. Optionally, 15 grams of orange flavor or 15 grams raspberry flavor is added. Optionally, 1000 grams of almonds, pecans or other nuts can be added. Pour mixture into a candy mold or pliable tray. Refrigerate until solidified.

### Example 11. Low GI Syrup

Ingredients: Psicose, Tagatose, Water, Isomaltulose, Trehalose, Natural and Artificial Flavor, Malic Acid, Xanthan Gum, Sodium Chloride, Sodium Hexametaphosphate, Preservatives (Sodium Benzoate, Sorbic Acid), Caramel Color, Phosphoric Acid, Pectin.

Method: 500 grams of psicose and 500 grams of tagatose are added to a heating container followed by 495 grams isomaltulose and 495 grams trehalose. Psicose, tagatose, isomaltulose, and trehalose are then blended together with 10 grams of xanthan gum until homogeneous. To the mixture is then added 700 grams water. The solution is heated to the boiling point and malic acid added. To the boiling solution is added 2 grams sodium chloride followed by 1.5 grams each of sodium hexametaphosphate, sodium benzoate and potassium sorbate. 0.85 grams of phosphoric acid is added to the solution. The system is heated until a Brix level of 74 is reached, removal from heat, and then followed by the addition of 15 grams of maple flavoring and caramel color.

### Example 12. Low GI Cough Syrup

Ingredients: Psicose, Tagatose, Water, Isomaltulose, Trehalose, Natural and Artificial Flavor, Malic Acid, Xanthan Gum, Sodium Chloride, Sodium Hexametaphosphate, dextromethorphan, menthol, Preservatives (Sodium Benzoate, Sorbic Acid), Caramel Color, Phosphoric Acid, Pectin.

Method: 500 grams of psicose and 500 grams of tagatose are added to a heating container followed by 495 grams isomaltulose and 495 grams trehalose. Psicose, tagatose, isomaltulose, and trehalose are then blended together with 10 grams of xanthan gum until homogeneous. To the mixture is then added 700 grams water. The solution is heated to the boiling point and malic acid added. To the boiling solution is added 2 grams sodium chloride followed by 1.5 grams each of sodium hexametaphosphate, sodium benzoate and potassium sorbate. 0.85 grams of phosphoric acid is added to the solution. The system is heated until a Brix level of 74 is reached, removal from heat, and then followed by the addition of a mixture of cherry flavoring, red coloring, dextromethorphan and menthol.

### Example 13. Low GI Cough Drops

Ingredients: Psicose, Tagatose, Water, Isomaltulose, Trehalose, Malic Acid, Citric Acid, Menthol, Natural and Artificial Flavor, FD&C Red No. 40.

Method: 500 grams of psicose and 500 grams of tagatose are added to a heating container followed by 495 grams isomaltulose, 495 grams trehalose and 15 grams of malic acid. The components are blended together until homogeneous and 400 grams of water is added. The system is heated to a vigorous boil and water is removed until a Brix of 93 is reached at which time 8 grams of a slurry of FD&C red No. 40 is added. The heat is removed and 9 grams of cherry flavoring and 2grams of menthol is added. 2.5 - 5-gram shapes are formed from the hot mixture by pouring the hot liquid mixture into a mold and allowed to cool to room temperature.

### Example 14. Low GI Non-dairy Creamer

Ingredients: Psicose, Tagatose, Isomaltulose, Trehalose, Xanthan Gum, Malic Acid, Sodium Benzoate, Potassium Sorbate, High Lauric Vegetable Fat, Sodium Caseinate, Mono/diglyceride Emulsifier (Cremodon 250/20), Dipotassium Hydrogen Phosphate, Sodium Carbonate.

Method: A emulsion is prepared, by first dissolving 13.68 kg psicose, 13.68 kg tagatose, 13.54 kg trehalose, 13.54 kg isomaltulose and 7.0 kg sodium caseinate in 90.7 kg warm water at 50° C., followed by the addition of 3 kg of the emulsifier. To this was dissolved 2.45 kg of dipotassium hydrogen phosphate and 0.27 kg of sodium carbonate. A melted fat phase was prepared from high lauric coconut oil at the same time and kept at a temperature no higher than 50° C. 31 kg of the melted fat phase was then poured into the aqueous phase with agitation to cause emulsification, at a temperature no higher than 50° C. The resultant emulsion was then homogenized under a pressure of 4000 psi. To the solution is added 0.85 kg of almond flavor and color.

Optionally, the above emulsion is passed to a spray drier of conventional design with centrifugal pressure nozzles and dried to a powder of 2% moisture content. An inlet temperature of 400° F. and an outlet temperature of 200° F. was employed, to give somewhat less than 98 kg dried product.

### Example 15. Low GI snickerdoodle

Ingredients: 1 cup butter softened, 1 1/2 cups low GI sugar mixture including trehalose, psicose and isomaltulose mixture, 2 eggs, 2 3/4 cups flour, 2 tsp cream of tartar, 1 tsp baking soda, 1/2 tsp salt, 2 tbsp the low GI sugar mixture, 1 tsp cinnamon

Method: Mix together flour, cream of tartar, baking soda, and salt together. Set aside. Cream together sugar and butter. Add eggs and blend well. Add dry ingredients to wet ingredients and mix well. Shape dough into 1 inch balls and roll in the cinnamon-sugar mixture. Place 2 inches apart on ungreased cookie sheet. Bake for 8-10 minutes at 350 degrees.

### Example 16. Low GI New York Style Cheesecake

Ingredients: Crust (2.5 cups whole wheat flour, 1.5 teaspoons baking soda, 0.25 teaspoon ground cinnamon, 0.25 teaspoon salt, 0.75 cup butter, softened, 1.0 cup trulinose, 0.25 cup molasses, 1.0 tablespoon vanilla extract). Filling and cake (1.0 cup trulinose, 1.5 cups sour cream, 0.25 cup melted butter, 2 eggs, 2.0 teaspoons vanilla, 1 pound of cream cheese broken into pieces, 2 tablespoons melted butter).

Method: In a medium bowl, whisk together flour, baking soda, cinnamon, and salt. Set aside. In a standing mixer fit with the paddle attachment, or with an electric hand mixer, cream butter and sugar together. Reduce speed to low and add molasses and vanilla until combined. Increase speed to medium and beat until light and fluffy. Reduce speed to low and add flour mixture until incorporated and a soft dough form. Scrape dough onto a work surface and knead to form a ball. Divide in half. Preheat oven to 350 degrees. Pat one ball of dough into rectangle, sprinkle with flour, flip, and dust again, Roll the dough until roughly 15-by-1 1 inches and very thin. Bake until crackers and firm and darkened, about 20 minutes. Cool to room temperature. Chop the cracker into crumbs.

Blend the cracker crumbs, 0.25cup sugar, and 0.25 cup melted butter, and line the bottom of an 8- or 9-inch ungreased spring form pan. Blend the sour cream, 0.75-cup a low GI sweet composition (trehalose, psicose and isomaltulose ), eggs, and vanilla. Add the cream cheese. Blend until smooth. Pour 2 tablespoons melted butter through the top of the machine. Pour into the pan. Bake at 325F in the oven for 45 minutes. Then broil until the top begins to show attractive brown spots. Refrigerate for at least 4 hours before cutting and serving.

### Example 16. Evaluation of the sensory property of the low GI sweet composition vs sucrose

Round table evaluations were performed with 20 test panelists. Three low GI sweet compositions (test 1, 2, and 3) were compare with an equal amount of sucrose. Test 1 composition included allulose, trehalose, and isomaltulose; test 2 included allulose and tagatose; test 3 included allulose and isomaltulose. In a double blind design, the test and reference compositions were presented to the panelists as white powdery substance coded with two-letter codes.

The test samples were tested against the reference samples in the double-blind test for the panelists to determine similarity or difference between the test and references samples. Each panelist was served with samples at room temperature. After each testing, the panelist was asked to rinse with room temperature water and wait for 15 minutes to clear their palates before testing the next sample.

The panelist rated the four basic taste profile (sweet, bitter, sour, metallic), intensity of each taste, the intensities of off-flavors (such as bitter, metallic and acidic) and the temporal profile of the sample (such as lingering after taste and the length of the linger). The length of the lingering was rated over a time period of 0 to 120 seconds. The results showed that there is statistically no different between the test compositions and reference sucrose in both the taste profile and the temporal profile of the sweetness. In addition, the results showed that the test samples lack the lingering "sour" note from sucrose. The length of the lingering from the test samples is shorter than that of sucrose sample. Specifically, the test sample 1 had the length of lingering from about 0 (instantaneous) to about 30 seconds. In comparison, the sucrose sample has a lingering "sour" taste lasting as long as from about 60 to about100 seconds.

## Claims

1. A sweet composition, comprising a low glycemic index sugar component having a glycemic index of not more than 20, wherein the sweet composition comprises not less than 50% weight of the low glycemic index sugar component, wherein the sweet composition is substantially free of D-sucrose, D-fructose, D-glucose, sugar alcohols, and non-sugar sweeteners, wherein the low glycemic index sugar component consists essentially of psicose, isomaltulose, and a third low glycemic index sugar selected from a group consisting of trehalose, sorbose, tagatose, or a combination thereof, and wherein isomaltulose and psicose are in a ratio of from 3:1 to 1:3.

2. The sweet composition of Claim 1, wherein the low glycemic index sugar component is not less than 90% weight of the sweet composition.

3. The sweet composition of Claim 1, wherein the low glycemic index sugar component consists of psicose, isomaltulose, and a third low glycemic index sugar selected from a group consisting of trehalose, sorbose, tagatose, or a combination thereof.

4. The sweet composition of Claim 1, wherein:
(i) trehalose and isomaltulose are in a ratio of from 3:1 to 1:3;
(ii) trehalose and psicose are in a ratio of from 3:1 to 1:3; and/or
(iii) psicose and tagatose are in a ratio of from 5:1 to 1:5.

5. The sweet composition of Claim 1, further comprising N-acetyl glucosamine.

6. The sweet composition of Claim 1, further comprising an active component, wherein the active component comprises a vitamin composition, a mineral composition, an amino acid composition, an antioxidant composition, an herbal composition, or an active pharmaceutical ingredient (API) composition.

7. The sweet composition of Claim 1, further comprising an additive, wherein the additive comprises a food acid, a flavoring agent, a coloring agent, a humectant, a bulking agent, a dispersant, an emulsifier, a thickener, a carbonation agent, or a combination thereof.

8. A powdered drink concentrate, comprising the sweet composition of Claim 1.

9. The powdered drink concentrate of Claim 8, further comprising a vitamin composition, a mineral composition, an herbal composition, a fruit extract, a carbonation agent, an antioxidant, a food acid, a dispersant, a flavoring agent, a coloring agent, or a combination thereof.

10. A syrup, comprising not less than 50% of the sweet composition of Claim 1.

11. The syrup of Claim 10, further comprising an active pharmaceutical ingredient, a flavoring agent, a coloring agent, or a combination thereof.

12. A confectionary product, comprising not less than 50% of the sweet composition of Claim 1.

13. A liquid composition, comprising not less than 5% of the sweet composition of Claim 1, a vitamin composition, a mineral composition, an active pharmaceutical ingredient, or a combination thereof.

14. A food product, comprising the sweet composition of Claim 1, wherein the food product comprises a gum, a chewable tablet, a popsicle, an ice cream, a milk product, a milk shake, a fruit juice, a coffee product, a soy milk product, yogurt, frozen yogurt, a slurpee, a liquid drink, a coffee mate, a carbonated drink, a soft drink, a soda, or a cooking mix.

15. The food product of Claim 14, wherein the food product comprises a gum or chewable tablet, and wherein the gum or chewable tablet comprises nicotine or caffeine.

## Patentansprüche

1. Eine süße Zusammensetzung, die eine Zuckerkomponente mit niedrigem glykämischem Index umfasst, die einen glykämischen Index von nicht mehr als 20 aufweist, wobei die süße Zusammensetzung zu nicht weniger als 50 Gewichts-% die Zuckerkomponente mit niedrigem glykämischem Index umfasst, wobei die süße Zusammensetzung im Wesentlichen frei von D-Saccharose, D-Fruktose, D-Glukose, Zuckeralkoholen und Nicht-Zucker-Süßstoffen ist, wobei die Zuckerkomponente mit niedrigem glykämischem Index im Wesentlichen aus Psicose, Isomaltulose und einem dritten Zucker mit niedrigem glykämischem Index, ausgewählt aus einer Gruppe, bestehend aus Trehalose, Sorbose, Tagatose oder einer Kombination davon, besteht, und wobei Isomaltulose und Psicose in einem Verhältnis von 3:1 bis 1:3 stehen.

2. Süße Zusammensetzung nach Anspruch 1, wobei die Zuckerkomponente mit niedrigem glykämischem Index nicht weniger als 90 Gewichts-% der süßen Zusammensetzung ausmacht.

3. Süße Zusammensetzung nach Anspruch 1, wobei die Zuckerkomponente mit niedrigem glykämischem Index aus Psicose, Isomaltulose und einem dritten Zucker mit niedrigem glykämischem Index, ausgewählt aus einer Gruppe, bestehend aus Trehalose, Sorbose, Tagatose oder einer Kombination davon, besteht.

4. Süße Zusammensetzung nach Anspruch 1, wobei:
(i) Trehalose und Isomaltulose in einem Verhältnis von 3:1 bis 1:3 stehen;
(ii) Trehalose und Psicose in einem Verhältnis von 3:1 bis 1:3 stehen; und/oder
(iii) Psicose und Tagatose in einem Verhältnis von 5:1 bis 1:5 stehen.

5. Süße Zusammensetzung nach Anspruch 1, die ferner N-Acetylglucosamin umfasst.

6. Süße Zusammensetzung nach Anspruch 1, die ferner eine aktive Komponente umfasst, wobei die aktive Komponente eine Vitaminzusammensetzung, eine Mineralzusammensetzung, eine Aminosäurezusammensetzung, eine Antioxidanszusammensetzung, eine pflanzliche Zusammensetzung oder eine arzneilich-wirksamer-Bestandteil(API)-Zusammensetzung umfasst.

7. Süße Zusammensetzung nach Anspruch 1, die ferner ein Additiv umfasst, wobei das Additiv ein Säuerungsmittel, einen Geschmacksstoff, einen Farbstoff, ein Feuchthaltemittel, einen Füllstoff, ein Dispersionsmittel, einen Emulgator, ein Verdickungsmittel, ein Karbonisierungsmittel oder eine Kombination davon umfasst.

8. Ein Pulvergetränkkonzentrat, das die süße Zusammensetzung nach Anspruch 1 umfasst.

9. Pulvergetränkkonzentrat nach Anspruch 8, das ferner eine Vitaminzusammensetzung, eine Mineralzusammensetzung, eine pflanzliche Zusammensetzung, einen Fruchtextrakt, ein Karbonisierungsmittel, ein Antioxidans, ein Säuerungsmittel, ein Dispersionsmittel, einen Geschmacksstoff, einen Farbstoff oder eine Kombination davon umfasst.

10. Ein Sirup, der zu nicht weniger als 50 % die süße Zusammensetzung nach Anspruch 1 umfasst.

11. Sirup nach Anspruch 10, der ferner einen arzneilich wirksamen Bestandteil, einen Geschmacksstoff, einen Farbstoff oder eine Kombination davon umfasst.

12. Ein Süßwarenprodukt, das zu nicht weniger als 50% die süße Zusammensetzung nach Anspruch 1 umfasst.

13. Eine flüssige Zusammensetzung, die zu nicht weniger als 5% die süße Zusammensetzung nach Anspruch 1, eine Vitaminzusammensetzung, eine Mineralzusammensetzung, einen arzneilich wirksamen Bestandteil oder eine Kombination davon umfasst.

14. Ein Lebensmittelprodukt, das die süße Zusammensetzung nach Anspruch 1 umfasst, wobei das Lebensmittelprodukt ein Kaugummi, eine kaubare Tablette, ein Stieleis, eine Eiscreme, ein Milchprodukt, einen Milchshake, einen Fruchtsaft, ein Kaffeeprodukt, ein Sojamilchprodukt, Joghurt, gefrorenen Joghurt, ein Slurpee, ein flüssiges Getränk, ein Coffee Mate, ein kohlensäurehaltiges Getränk, einen Softdrink, ein Soda oder eine Kochmischung umfasst.

15. Lebensmittelprodukt nach Anspruch 14, wobei das Lebensmittelprodukt ein Kaugummi oder eine kaubare Tablette umfasst und wobei das Kaugummi oder die kaubare Tablette Nikotin oder Koffein umfasst.

## Revendications

1. Composition sucrée, comprenant un composant sucre à faible indice glycémique ayant un indice glycémique ne dépassant pas 20, la composition sucrée comprenant au moins 50 % en poids du composant sucre à faible indice glycémique, la composition sucrée étant sensiblement exempte de D-saccharose, de D-fructose, de D-glucose, de sucres alcools et d'édulcorants sans sucre, le composant sucre à faible indice glycémique consistant essentiellement en psicose, en isomaltulose, et en un troisième sucre à faible indice glycémique sélectionné dans un groupe constitué du tréhalose, du sorbose, du tagatose, ou d'une combinaison de ceux-ci, et l'isomaltulose et le psicose étant présents selon un rapport de 3:1 à 1:3.

2. Composition sucrée selon la revendication 1, dans laquelle le composant sucre à faible indice glycémique constitue au moins 90 % en poids de la composition sucrée.

3. Composition sucrée selon la revendication 1, dans laquelle le composant sucre à faible indice glycémique consiste en psicose, en isomaltulose, et en un troisième sucre à faible indice glycémique sélectionné dans un groupe constitué du tréhalose, du sorbose, du tagatose, ou d'une combinaison de ceux-ci.

4. Composition sucrée selon la revendication 1, dans laquelle :
(i) le tréhalose et l'isomaltulose sont présents selon un rapport de 3:1 à 1:3 ;
(ii) le tréhalose et le psicose sont présents selon un rapport de 3:1 à 1:3 ; et/ou
(iii) le psicose et le tagatose sont présents selon un rapport de 5:1 à 1:5.

5. Composition sucrée selon la revendication 1, comprenant en outre de la N-acétylglucosamine.

6. Composition sucrée selon la revendication 1, comprenant en outre un composant actif, le composant actif comprenant une composition vitaminique, une composition minérale, une composition d'acides aminés, une composition antioxydante, une composition à base de plantes, ou une composition qui est un principe actif pharmaceutique (PAP).

7. Composition sucrée selon la revendication 1, comprenant en outre un additif, l' additif comprenant un acide alimentaire, un aromatisant, un colorant, un humectant, un agent de masse, un dispersant, un émulsifiant, un épaississant, un agent de carbonatation, ou une combinaison de ceux-ci.

8. Concentré de boisson en poudre, comprenant la composition sucrée selon la revendication 1.

9. Concentré de boisson en poudre selon la revendication 8, comprenant en outre une composition vitaminique, une composition minérale, une composition à base de plantes, un extrait de fruit, un agent de carbonatation, un antioxydant, un acide alimentaire, un dispersant, un aromatisant, un colorant, ou une combinaison de ceux-ci.

10. Sirop, comprenant au moins 50 % de la composition sucrée selon la revendication 1.

11. Sirop selon la revendication 10, comprenant en outre un principe actif pharmaceutique, un aromatisant, un colorant, ou une combinaison de ceux-ci.

12. Produit de confiserie, comprenant au moins 50 % de la composition sucrée selon la revendication 1.

13. Composition liquide, comprenant au moins 5 % de la composition sucrée selon la revendication 1, une composition vitaminique, une composition minérale, un principe actif pharmaceutique, ou une combinaison de ceux-ci.

14. Produit alimentaire, comprenant la composition sucrée selon la revendication 1, le produit alimentaire comprenant un chewing-gum, un comprimé à croquer, une sucette glacée, une glace, un produit laitier, un milk-shake, un jus de fruit, un produit au café, un produit au lait de soja, un yaourt, un yaourt glacé, un slurpee, une boisson liquide, un coffee mate, une boisson gazeuse, une boisson sans alcool, un soda, ou un mélange culinaire.

15. Produit alimentaire selon la revendication 14, le produit alimentaire comprenant un chewing-gum ou un comprimé à croquer, et le chewing-gum ou le comprimé à croquer comprenant de la nicotine ou de la caféine.
